# EUROPEAN PATENT APPLICATION

(11) **EP 4 663 098 A1**
(43) Date of publication of application: **17.12.2025**
(21) Application number: 24461584.5
(22) Date of filing: 13.06.2024
(51) Int. Cl.: A61B 3/10

(54) **METHOD AND DEVICE FOR PRECISE MEASUREMENT OF OCULAR BIOMETRIC PARAMETERS BY MEANS OF OPTICAL COHERENCE TOMOGRAPHY (OCT)**

(71) Applicant: OPTOPOL Technology Spolka z ograniczona Odpowiedzialnoscia, 42-400 Zawiercie (PL)
(72) Inventor: BEDNARCZYK, Grzegorz, 42-400 Zawiercie (PL); SLUSARCZYK, Grzegorz, 40-213 Katowice (PL); STANISZEWSKI, Pawel, 42-480 Poreba (PL); WOJDAS, Lukasz, 42-400 Zawiercie (PL)
(74) Representative: AOMB Polska Sp. z.o.o.

(57) **Abstract**

The invention relates to a method and device for precise measurement of ocular biometric parameters by means of optical coherence tomography (OCT). This system consists of a mobile OCT head, which generates and records optical waves, a reference path with a controlled displacement mechanism, a measurement path and two off-axial cameras for determining the three-dimensional position of the centre of the pupil in the eye undergoing examination. The proposed method and device are programmed to perform a series of measurement stages, from setting the head and adjusting it to the optimal distance from the eye, on to precise determination of the position of the reference path and focus, and up to detailed scanning of the structures of the eye. The invention offers an improvement in the precision of ophthalmic diagnostics, which has considerable impact on healing and monitoring the state of health of the eyes.

## Description

The object of the invention is a method for determining ocular biometric parameters, which uses advanced technologies of optical coherence tomography (OCT) for precise and non-invasive scanning of the inner structures of the eye.

The development of biometric methods for measuring the axial length of the human eye, which are crucial for diagnostics and for planning ophthalmic surgeries, began as early as in 1986. It was then that Fercher and Roth for the first time presented the application of laser interferometry for this purpose. Progress in this field resulted in commercial introduction of devices such as IOLMaster 500 from the Carl Zeiss Meditec company, which use partial coherence interferometry (PCI), increasing the precision of measuring the anterior chamber depth (ACD) and the lens thickness (LT) considerably. Major breakthroughs also occurred with the introduction of the Optical Coherence Tomography (OCT) technology, including the Swept Source OCT version (SS-OCT), which offer even higher precision and diagnostic possibilities. Not only do devices using this technology, such as IOL Master 700 from Carl Zeiss Meditec and Movu from the Argos company, measure the axial length of the eye precisely, but they also enable detailed imaging of the retina and the optic nerve, which is invaluable when diagnosing and healing posterior eye segment diseases. The latest innovations, like the split window (SW-OCT), presented by Sikorski and Suchon in the B-OCT method, open up new prospects for measuring the axial dimensions of the eye with even greater precision. Since the first attempts at using optical measurement methods in the 1980s, biometric techniques have made much progress, transforming the manner in which specialists may measure and analyse the structures of the eye. Not only did the commercial introduction of advanced devices which use modern technologies, such as OCT, improve the precision of measurements, but it also considerably expanded diagnostic possibilities in ophthalmology. In spite of this progress, there are remaining challenges, especially in the context of transparency of the eye undergoing examination, which can affect the results of measurements. Further progress and optimisation of the OCT technology and other biometric methods will be crucial for future innovations in this field.

European patent application EP4226842A1 discloses an advanced ophthalmic apparatus, which integrates the functionalities of an eye fundus camera and an OCT device (optical coherence tomography). This apparatus is meant to diagnose and analyse the eye fundus, offering detailed examinations using the OCT technology. This device allows for the acquisition of high-resolution cross-sectional images of the structures of the eye, thus enabling precise assessment of the condition of the retina, the optic nerve, and other structures of the eye. The OCT technology used in the apparatus provides images with micrometre-level precision, non-invasively. It is widely used in diagnostics of diseases such as macular degeneration, retinal detachment or glaucoma, as well as to monitor the progress of a disease and the effects of treatment. The apparatus uses various modalities of OCT, such as swept-source OCT, spectral domain OCT and TD-OCT, enabling the adjustment of examinations to individual diagnostic needs. The document also emphasises the role of encoders in precise positioning and control of the mechanical movement of components in the device, which is crucial for retaining imaging quality and the precision of scanning. Encoders allow for tracking the position of the mobile parts of the apparatus, thus enabling precise setting and scanning of specific areas in the eye. The device allows for measuring such dimensions of the eye as the thickness of retinal layers, the thickness of the nerve fibre layer in the optic nerve, measuring the area and volume of the vitreous body, the topography and morphology of the optic nerve head, as well as for mapping and analysing the microstructure of the anterior eye segment.

Document US20220338731A1 describes an advanced measurement system used in ophthalmology, which integrates optical coherence tomography (OCT) and specialised encoders for precise positioning of optical elements. This system is found useful mainly in diagnostics and planning of ophthalmic surgeries, including those of cataracts. The key element is a Stokes cell, operated by two cylinder lenses, which may be both plano-concave and plano-convex, rotated by means of stepper motors for correcting astigmatism. The encoders read the orientation of the lenses precisely, thus enabling adjustment of the optical system to the patient's needs. The OCT technology allows for obtaining high-resolution cross-sections of the eye, thus supporting precision of measurements. This device also integrates other technologies, such as wavefront aberrometry and corneal topography, thus providing comprehensive evaluation of the condition of the eye. A fixation target system controls the patient's accommodation and line of sight during the examination, which is critical for the precision of measurements. The device offers a tool for evaluating the condition of eye refraction, necessary to diagnose and cure eye diseases. It performs biometric measurements, including those of the central corneal thickness (CCT), the anterior chamber depth (ACD), the pupil diameter (PD), the white-to-white distance (WTW), the lens thickness (LT), the axial length (AL), and the retinal layer thickness.

Patent description EP3659494B1 presents an advanced ophthalmic system, which uses a plurality of ophthalmic imaging apparatuses, such as slit lamp microscopes and OCT apparatuses, to perform detailed ophthalmic examinations. This system integrates artificial intelligence technologies (AI) to analyse three-dimensional images of the eye, intended to support the processes of diagnosing and monitoring eye conditions. A critical role in this system is served by an encoder, which precisely detects and monitors the focal position and the direction of imaging and illumination apparatuses. This is extremely important for ensuring the precision and reproducibility of the performed ophthalmic examinations.

Document WO2023161272A1 describes a computer-implemented method for biometric analysis of the human eye, which is based on image data acquired by means of optical coherence tomography (OCT). The main purpose of this method is identification and segmentation of the structures of the eyes, such as the cornea, the lens (natural or artificial) and the retina, on the basis of OCT images. A key aspect of this invention is the use of an algorithm based on artificial intelligence, in particular neural networks, such as U-Net, to create a probability map for each pixel of the image data. This map indicates whether the respective pixel can be assigned to an interface of an eye structure. The invention describes a method for training the AI algorithm on the basis of manually labelled data, which precisely determine the boundaries of structures of the eye in OCT images. Due to the application of AI for an analysis of OCT images, the method can automatically and precisely identify the structures of the eye, which is particularly useful in cases which are difficult to analyse, e.g. eyes with artificial lenses (IOL).

In consideration of the above limitations of the available prior art, the purpose of the invention is to considerably improve the precision and efficiency of ophthalmic diagnostics by developing a method for determining ocular biometric parameters. The invention uses a mobile head for optical coherence tomography (OCT), which allows for precise generation and recording of optical waves, enabling detailed examination of various structures of the eye. The application of advanced scanning techniques and positioning algorithms allows for producing more precise and reproducible results, which is necessary to improve medical standards. The invention also offers complex imaging capabilities, enabling precise analyses of the cornea, the lens and the retina with high resolution. Due to the use of two cameras and advanced procedures, such as "Auto C-gate", "Auto Focus" and the tracking technique, the invention minimises the risk of human errors and automates the measurement process, which translates into quicker and more effective examinations. In addition, the invention integrates the collected data in order to create a detailed database, which may be used to monitor the state of health of the patient's eyes over time, providing physicians with valuable information, necessary for effective healing.

The object of the invention is a method for determining ocular biometric parameters, implemented by means of a device comprising a mobile head for optical coherence tomography, OCT, capable of generating and recording optical waves, a reference path with a controlled displacement mechanism, a measurement path, two cameras placed off-axially with respect to the OCT optical axis to determine the position of the centre of the pupil in the eye undergoing examination in three dimensions X, Y, Z, a processor, memory and software for processing data and generating OCT images on the basis of the data recorded by the OCT head and the data describing the reference path, comprising an initial stage and a main stage, wherein the initial stage comprises the following steps:
a) setting the head to a default position for Posterior examinations, and detecting the pupil by means of said two cameras; moving the OCT head towards the eye to a predefined distance from the cornea, which results in a change in the eye-lens distance in the Z axis, preferably to a distance of 37 mm, and setting the OCT head in the ocular axis;
b) finding a proper position of the reference path, meaning a difference in the distance between the reference path and the retina of the eye undergoing examination, which is smaller than the imaging range of the device along the OCT optical axis, preferably by means of the "Auto C-gate" procedure, by changing the length of the reference path and scanning the retina of the eye undergoing examination; finding an optimal value of "focus", meaning the position of the lens, in which the axial defect of the eye undergoing examination is compensated for, preferably by means of the "Auto Focus" procedure; setting the signal in the middle of the imaging window by means of an image analysis of tomograms, and using the movements of the device head in the X, Y, Z axes, preferably by means of the "tracking" procedure, and performing shadow correction by moving the OCT head;
c) remembering the position of the OCT head, the position of the reference path, and the optimal value of focus, for which the signal has been set;
d) setting the head to a default position for Anterior examinations, and detecting the pupil; moving the OCT head towards the eye to a predefined distance from the cornea, which results in a change in the eye-lens distance in the Z axis, preferably by 52 mm or 56 mm, and setting the OCT head in the ocular axis;
e) finding a proper position of the reference path, meaning a difference in the distance between the reference path and the lens of the eye undergoing examination, which is smaller than the imaging range of the device along the OCT optical axis, as well as the position of the upper edge of the lens, preferably by means of the "Auto C-gate" procedure; finding an optimal value of "focus", meaning the position of the lens, in which the axial defect of the eye undergoing examination is compensated for, preferably by means of the "Auto Focus" procedure; positioning the cornea in the middle of the tomogram, preferably by means of the "tracking" procedure, preferably XY "tracking";
f) calculating four positions of the reference path and an optimal value of focus for the cornea, for the front edge of the lens, for the back edge of the lens, and for the retina, on the basis of the position of the front part of the lens of the eye undergoing examination, wherein the back position of the lens of the eye undergoing examination is calculated on the basis of the position of the front part of the lens and a constant value of 4 mm;
   whereas the main stage comprises the following steps:
g) setting the reference path and focus in a position for the cornea; performing a horizontal and vertical scan of the cornea in the eye undergoing examination; remembering the position of the reference path;
h) setting the reference path and focus in a position for the front edge of the lens; performing a horizontal and vertical scan of the front edge of the lens; remembering the position of the reference path;
i) setting the reference path and focus in a position for the edge of the retina; performing a horizontal and vertical scan of the edge of the retina; remembering the position of the reference path;
j) repeating the setting of the reference path and focus in a position for the retina, and performing scans like in steps g) to i);
k) repeating the setting of the reference path and focus in a position for the back edge of the lens, and performing scans like in steps g) to j);
l) repeating the setting of the reference path and focus in a position for the front edge of the lens, and performing scans like in steps g) to k);
wherein the results of individual steps g) to l) are saved in a database as tomograms along with their corresponding position of the reference path; and subsequently, the ocular biometric parameters are calculated on the basis of the data acquired during the initial stage and during the main stage.

Preferably, the method is characterised in that it uses a device comprising an encoder to measure the position of the reference path, while the position of the reference path mentioned in steps from g) to l) is a position read from the encoder. Preferably, the method is characterised in that the determination whether the patient has a natural lens or an intraocular lens, IOL, is made in step a) at the latest. Preferably, the method is characterised in that the cycle of steps from g) to l) is repeated 10 times. Preferably, the method is characterised in that the ocular biometric parameters are calculated using the position of the reference path, and an analysis of the OCT image.

Preferably, the method is characterised in that the ocular biometric parameters are calculated using proper refractive indices of light for specific layers of the eye. Preferably, the method is characterised in that the ocular biometric parameters are calculated using artificial intelligence algorithms.

Preferably, the method is characterised in that it determines one or more of the following parameters: the central corneal thickness, CCT; the anterior chamber depth, ACD; the lens thickness, LT; the axial length of the eye, AL. Preferably, the method is characterised in that it uses a portable device.

The object of the invention is also a device for determining ocular biometric parameters, using the optical coherence tomography technique (OCT), comprising a mobile head for optical coherence tomography (OCT), capable of generating and recording optical waves, a reference path with a controlled displacement mechanism, a measurement path, two cameras placed off-axially with respect to the OCT optical axis to determine the position of the centre of the pupil in the eye undergoing examination in three dimensions X, Y, Z, a processor, memory and software for processing data and generating OCT images on the basis of the data recorded by the OCT head and the data describing the reference path, configured and programmed to implement the method for determining ocular biometric parameters.

Preferably, the reference path of the device has a moving mirror driven by a stepper motor via a gear with a rack, and a mirror placed on a linear bearing which enables motion.

Preferably, the device additionally has an encoder, preferably magnetic or optical, preferably incremental or absolute, for measuring the position of the reference path. Preferably, the encoder of the device is configured to measure the displacement of the reference path between measurement windows.

Preferably, the encoder of the device is magnetic, the magnetic tape of the encoder is placed on a moving carriage along with the mirror, and the encoder sensor is placed on a frame shared with the rail of the linear bearing. The device is a portable device.

The present invention, describing a method and device for precise measurement of ocular biometric parameters by means of optical coherence tomography (OCT), entails a number of benefits. First of all, this method does not require the use of invasive or dangerous examination techniques, thus increasing the safety of the patients and the medical personnel. Due to the use of advanced software and a precisely controlled OCT head, not only is the measurement process quick, but also highly precise, thus allowing for the generation of reproducible biometric images of high quality. In addition, due to the stage of automatic adjustment of settings of the head and the reference path, it is possible to produce optimal measurement results in a shorter time, thus considerably shortening the total time of examination according to claim 1. This invention also provides better adjustment to the patients' individual needs due to the use of procedures such as "tracking" and "Auto Focus", which allow for precise adjustment of scanning parameters to a specific case.

The use of an encoder in the method and device for precise measurement of ocular biometric parameters by means of optical coherence tomography (OCT) provides considerable benefits. Being a device measuring a displacement, an angle or a position, the encoder is a critical component which increases the precision and reproducibility of measurements, and it is responsible for measuring the displacement of the reference path. The encoder allows for precise tracking of the movements of the mobile OCT head, which is necessary to precisely position the device with respect to the patient's eye. Precise tracking of the movements of the head allows for maintaining a constant distance and orientation with respect to the eye undergoing examination, which is important for producing credible measurement results. Automatic control of the position of the OCT head by means of the encoder minimises the need for manual corrections and interventions of an operator, which translates into higher efficiency and speed of the performed examinations. The encoder ensures automatic and reproducible achievement of the desired settings, which is particularly important in examinations which require repeated measurements of the same parameters. By maintaining precise control over the movements of the head, the encoder helps to acquire stable and high quality OCT images. This stability is critical, since even small vibrations or an improper setting may distort the image, and therefore affect the precision of diagnosis. Precise and quick measurements enable better adaptation of the examination procedures to the patients' individual needs.

It is important to determine whether the patient has a natural lens or an intraocular lens (IOL), since it affects the planning and adjustment of diagnostic and surgical procedures. The differences in optical properties between natural lenses and IOLs may require changing the settings of the measurement devices, such as coherence tomography (OCT), in order to ensure precision of measurements.

The measurement of parameters such as the central corneal thickness (CCT), the anterior chamber depth (ACD), the lens thickness (LT), and the axial length of the eye (AL) is highly preferable in many aspects of diagnosing and monitoring the health of the eyes. Each of these parameters provides crucial information which is applicable to various areas of ophthalmology. The measurement of the CCT is important in diagnosing and monitoring glaucoma, since corneal thickness may affect the precision of measuring intraocular pressure (IOP), which is a key factor in managing and assessing the risk of development of glaucoma. The measurement of the ACD is important in planning cataract surgeries, since it allows for precise adjustment of the power of an artificial intraocular lens (IOL). Information about the lens thickness is important, particularly in the context of assessing the condition of the lens, for example for the development of cataracts. The axial length of the eye is a fundamental measurement for determining eye refraction, as well as for diagnosing and managing myopia and hyperopia.

The use of artificial intelligence algorithms (Al) for the calculation of ocular biometric parameters provides a number of benefits. Artificial intelligence can speed up the data analysis process, enabling quicker performance of the examinations, and shortening the waiting time for the patients.

In addition, the universality of the measurement algorithm, which is used in the device for precise measurement of ocular biometric parameters by means of optical coherence tomography (OCT), constitutes one of the key advantages of this invention. This feature allows for using the algorithm in various configurations of OCT devices, which extends its applicability in medical practice considerably.

The device whose reference path has a moving mirror driven by a stepper motor via a gear with a rack, and a mirror placed on a linear bearing which enables motion, ensures high reproducibility and replicability of the measurements in such an arrangement of the technical elements.

The portable device for determining the ocular biometric parameters which uses the optical coherence tomography technique (OCT) provides the flexibility of diagnostics in various environments, from clinics to hospital wards and field health care centres, allowing access to ophthalmic examinations in places where large permanent installations would be infeasible or uneconomic.

The invention will be presented more closely in the following preferable embodiments not limiting the scope of the invention, with reference to the attached figures, in which:

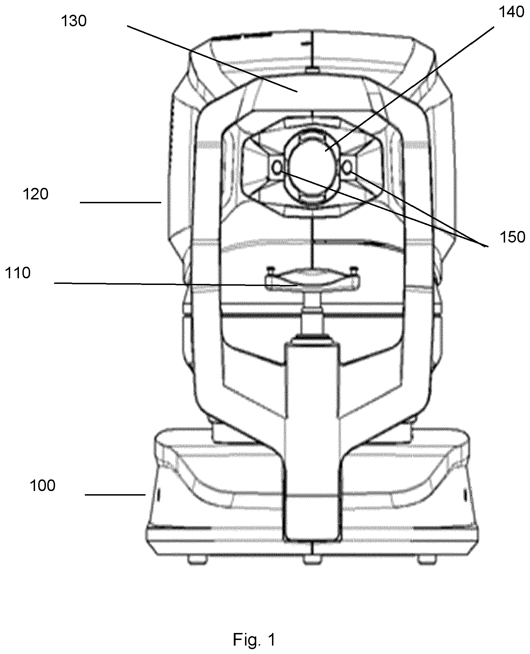

**Fig. 1** presents a general view of the device, where the following references represent:
   100-a device base,
   110-a chinrest for the patient,
   120-a device head,
   130-a forehead rest for the patient,
   140-an objective lens,
   150-two cameras placed off the OCT axis.

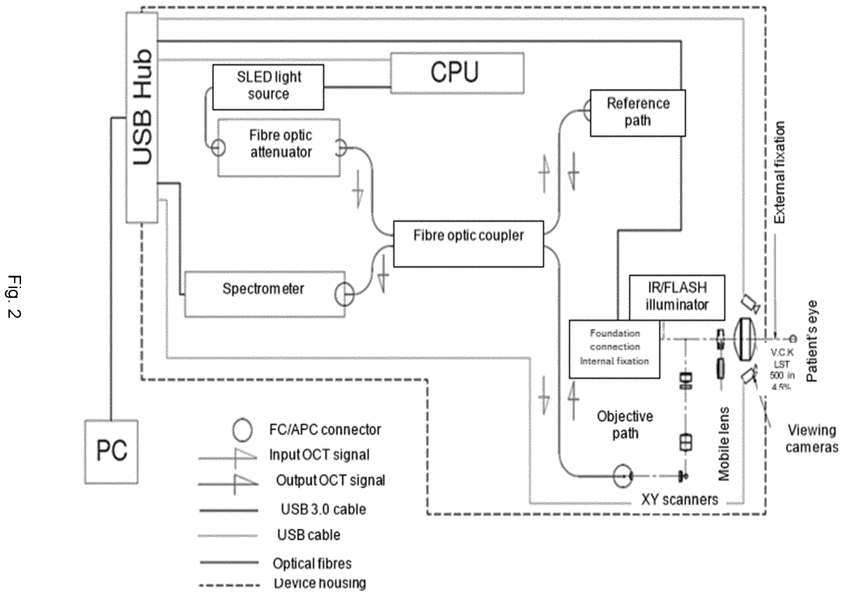

**Fig. 2** presents a diagram of the device head in which there is a reference path, with an encoder installed therein, due to which the reproducibility and replicability of the measurements of biometric parameters have been improved.
**Fig. 3** presents an embodiment of the reference path with a mounted encoder, where the following references represent:
   200-optical fibre,
   210-collimator lens,
   220-moving mirrors,
   230-linear bearing,
   240-measurement tape of the magnetic encoder,
   250-rack,
   260-stepper motor,
   270-gear wheel,
   280-encoder,
   290-retroreflector lens,
   300-retroreflector mirror.

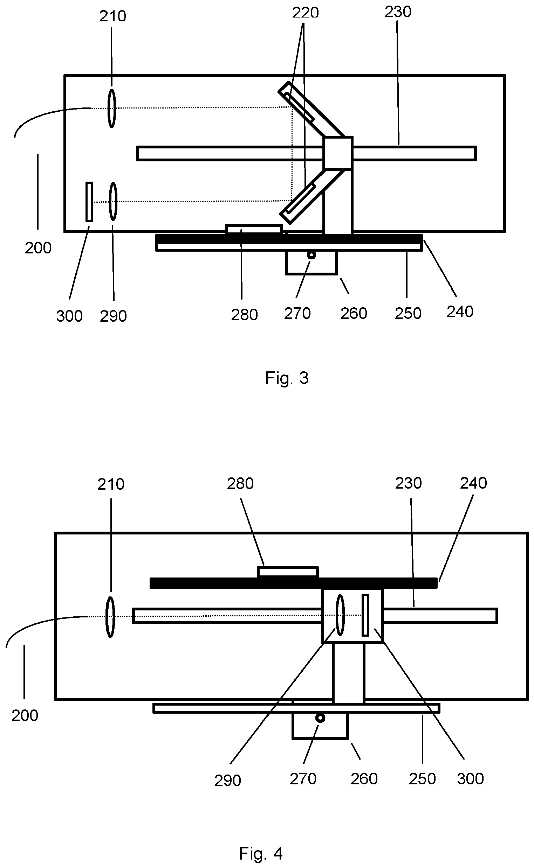

Figure 3 discloses a method for assembling the encoder 280 and the measurement tape 240 so as to minimise the impact of mechanical inaccuracies on measurement results.
**Fig. 4** presents an embodiment of the reference path with a mounted encoder and a single moving mirror, where the following references represent:
   200-optical fibre,
   210-collimator lens,
   230-linear bearing,
   240-measurement tape of the magnetic encoder,
   250-rack,
   260-stepper motor,
   270-gear wheel,
   280-encoder,
   290-retroreflector lens,
   300-retroreflector mirror.

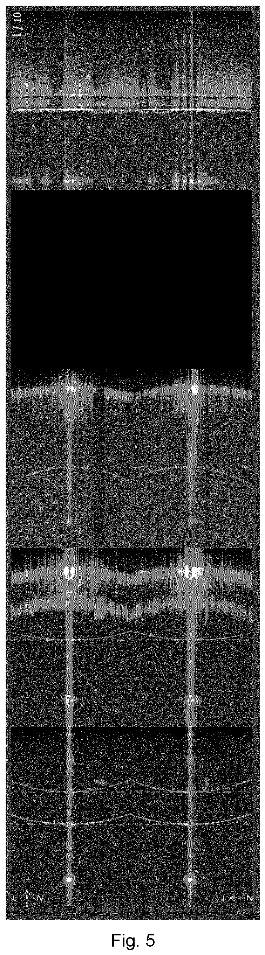

**Fig. 5** presents a sample measurement result for an artificial eye, bottom to top: the cornea, the lens, the retina.

The invention is presented in detail in the following embodiments. The embodiments do not limit the scope of the invention.

### Embodiment 1

Before separating the stage of adjusting the automatic adjustment of the settings of the head and the reference path, the average duration of 10 repetitions of biometrics is about 258 seconds. Due to the separation of the stage of automatic adjustment of the settings of the head and the reference path, the duration of the examination is reduced significantly, since a single stage of setting the head and the reference path is 25.8 seconds and 4 seconds per 10 repetitions of the examinations. 4 seconds when the patient must remain relatively still during the measurement and keep the eye open.

The dispersion (the difference between the maximum and minimum values) of the results for the biometric parameters AL, ACD, LT, CCT is reduced due to the use of an encoder.

The following tables present replicability measured for devices without the use of an encoder and with the use of an encoder, for individual biometric parameters AL, ACD, LT, CCT. A sample OCT result is also presented in Fig. 6. The calculated range at the bottom clearly indicates that, following the use of the encoder, the dispersion of the results is reduced, e.g. by a factor of 10 for AL, by a factor of 2 for ACD, and by a factor of 2.5 for LT.

**Table 1. Reproducibility measured between the devices for devices not using an encoder**

| **Device without an encoder** | **AL [mm]** | **ACD [mm]** | **LT [mm]** | **CCT [mm]** |
|---|---|---|---|---|
| #1 | 26.45 | 3.46 | 4.04 | 0.529 |
| #2 | 26.46 | 3.46 | 3.98 | 0.53 |
| #3 | 26.35 | 3.42 | 4.04 | 0.527 |
| #4 | 26.34 | 3.53 | 4.16 | 0.528 |
| #5 | 26.36 | 3.47 | 4.11 | 0.53 |
| #6 | 26.42 | 3.48 | 4.06 | 0.529 |
| #7 | 26.55 | 3.45 | 4.06 | 0.526 |
| #8 | 26.51 | 3.5 | 4.03 | 0.527 |
| #9 | 26.51 | 3.48 | 4.06 | 0.532 |
| #10 | 26.44 | 3.48 | 4.05 | 0.528 |
| | | | | |
| **Average values** | 26.439 | 3.473 | 4.059 | 0.5286 |
| **Standard deviation (Sd)** | **0.0688** | **0.0279** | **0.0455** | **0.0017** |
| **Dispersion** | **0.21** | **0.11** | **0.18** | **0.006** |

**Table 2. Reproducibility measured between the devices for devices using an encoder**

| **Device with an encoder** | **AL [mm]** | **ACD [mm]** | **LT [mm]** | **CCT [mm]** |
|---|---|---|---|---|
| #1 | 25.21 | 3.89 | 3.7 | 0.5 |
| #2 | 25.21 | 3.88 | 3.67 | 0.5 |
| #3 | 25.22 | 3.88 | 3.7 | 0.501 |
| #4 | 25.21 | 3.91 | 3.72 | 0.504 |
| #5 | 25.23 | 3.87 | 3.72 | 0.5 |
| #6 | 25.22 | 3.88 | 3.71 | 0.501 |
| #7 | 25.21 | 3.91 | 3.71 | 0.504 |
| #8 | 25.21 | 3.91 | 3.73 | 0.503 |
| #9 | 25.21 | 3.91 | 3.74 | 0.503 |
| #10 | 25.23 | 3.84 | 3.69 | 0.5 |
| | | | | |
| **Average values (Avg)** | 25.216 | 3.888 | 3.709 | 0.5016 |
| **Standard deviation (Sd)** | **0.0080** | **0.0218** | **0.0192** | **0.0016** |
| **Dispersion** | **0.02** | **0.07** | **0.07** | **0.004** |

The use of an encoder in biometric measurements, such as the axial length of the eye (AL), the anterior chamber depth (ACD), the lens thickness (LT) and the central corneal thickness (CCT), improves the precision and reproducibility of these measurements considerably. While comparing the tables before and after the use of the encoder, it is possible to come up with several key aspects related to its significance in the context of biometric measurements; namely, in the case of the axial length of the eye parameter (the AL dispersion), the dispersion of the results was reduced by a factor of ten, which indicates a considerable increase in precision. Analogically, in the case of the anterior chamber depth (ACD) and the lens thickness (LT), the dispersion was reduced by a factor of two and two and a half, respectively. Due to the use of the encoder, the acquired data are more coherent and reproducible, which is extremely important in precise diagnostics and therapy.

## Claims

1. A method for determining ocular biometric parameters, implemented by means of a device comprising a mobile head 120 for optical coherence tomography, OCT, capable of generating and recording optical waves, a reference path with a controlled displacement mechanism, a measurement path, two cameras 150 placed off-axially with respect to the OCT optical axis to determine the position of the centre of the pupil in the eye undergoing examination in three dimensions X, Y, Z, a processor, memory and software for processing data and generating OCT images on the basis of the data recorded by the OCT head and the data describing the reference path, comprising an initial stage and a main stage, wherein:
the initial stage comprises the following steps:
a) setting the head to a default position for Posterior examinations, and detecting the pupil by means of said two cameras 150; moving the OCT head 120 towards the eye to a predefined distance from the cornea, which results in a change in the eye-lens distance in the Z axis, preferably to a distance of 37 mm, and setting the OCT head in the ocular axis;
b) finding a proper position of the reference path, meaning a difference in the distance between the reference path and the retina of the eye undergoing examination, which is smaller than the imaging range of the device along the OCT optical axis, preferably by means of the "Auto C-gate" procedure, by changing the length of the reference path and scanning the retina of the eye undergoing examination; finding an optimal value of "focus", meaning the position of the lens, in which the axial defect of the eye undergoing examination is compensated for, preferably by means of the "Auto Focus" procedure; setting the signal in the middle of the imaging window by means of an image analysis of tomograms, and using the movements of the device head in the X, Y, Z axes, preferably by means of the "tracking" procedure, and performing shadow correction by moving the OCT head;
c) remembering the position of the OCT head, the position of the reference path, and the optimal value of focus, for which the signal has been set;
d) setting the head to a default position for Anterior examinations, and detecting the pupil; moving the OCT head towards the eye to a predefined distance from the cornea, which results in a change in the eye-lens distance in the Z axis, preferably by 52 mm or 56 mm, and setting the OCT head in the ocular axis;
e) finding a proper position of the reference path, meaning a difference in the distance between the reference path and the lens of the eye undergoing examination, which is smaller than the imaging range of the device along the OCT optical axis, as well as the position of the upper edge of the lens, preferably by means of the "Auto C-gate" procedure; finding an optimal value of "focus", meaning the position of the lens, in which the axial defect of the eye undergoing examination is compensated for, preferably by means of the "Auto Focus" procedure; positioning the cornea in the middle of the tomogram, preferably by means of the "tracking" procedure, preferably XY "tracking";
f) calculating four positions of the reference path and an optimal value of focus for the cornea, for the front edge of the lens, for the back edge of the lens, and for the retina, on the basis of the position of the front part of the lens of the eye undergoing examination, wherein the back position of the lens of the eye undergoing examination is calculated on the basis of the position of the front part of the lens and a constant value of 4 mm;
whereas the main stage comprises the following steps:
g) setting the reference path and focus in a position for the cornea; performing a horizontal and vertical scan of the cornea in the eye undergoing examination; remembering the position of the reference path;
h) setting the reference path and focus in a position for the front edge of the lens; performing a horizontal and vertical scan of the front edge of the lens; remembering the position of the reference path;
i) setting the reference path and focus in a position for the edge of the retina; performing a horizontal and vertical scan of the edge of the retina; remembering the position of the reference path;
j) repeating the setting of the reference path and focus in a position for the retina, and performing scans like in steps g) to i);
k) repeating the setting of the reference path and focus in a position for the back edge of the lens, and performing scans like in steps g) to j);
l) repeating the setting of the reference path and focus in a position for the front edge of the lens, and performing scans like in steps g) to k);
wherein the results of individual steps g) to l) are saved in a database as tomograms along with their corresponding position of the reference path;
and subsequently, the ocular biometric parameters are calculated on the basis of the data acquired during the initial stage and during the main stage.

2. The method according to claim 1, **characterised in that** it uses a device comprising an encoder to measure the position of the reference path, while the position of the reference path mentioned in steps from g) to l) is a position read from the encoder.

3. The method according to claim 1 or 2, **characterised in that** the determination whether the patient has a natural lens or an intraocular lens, IOL, is made in step a) at the latest.

4. The method according to any of the claims from 1 to 3, **characterised in that** the cycle of steps from g) to l) is repeated, preferably 10 times.

5. The method according to any of the claims from 1 to 4, **characterised in that** the ocular biometric parameters are calculated using the position of the reference path, and an analysis of the OCT image.

6. The method according to any of the claims from 1 to 5, **characterised in that** the ocular biometric parameters are calculated using proper refractive indices of light for specific layers of the eye.

7. The method according to any of the claims from 1 to 6, **characterised in that** the ocular biometric parameters are calculated using artificial intelligence algorithms.

8. The method according to any of the claims from 1 to 7, **characterised in that** it determines one or more of the following parameters: the central corneal thickness, CCT; the anterior chamber depth, ACD; the lens thickness, LT; the axial length of the eye, AL.

9. The method according to any of the claims from 1 to 8, **characterised in that** it uses a portable device.

10. A device for determining ocular biometric parameters, using the optical coherence tomography technique (OCT), comprising a mobile head 120 for optical coherence tomography (OCT), capable of generating and recording optical waves, a reference path with a controlled displacement mechanism, a measurement path, two cameras 150 placed off-axially with respect to the OCT optical axis to determine the position of the centre of the pupil in the eye undergoing examination in three dimensions X, Y, Z, a processor, memory and software for processing data and generating OCT images on the basis of the data recorded by the OCT head and the data describing the reference path, configured and programmed to implement the method according to any of the preceding claims from 1 to 9.

11. The device according to claim 10, whose reference path has a moving mirror 300 driven by a stepper motor 260 via a gear with a rack 250, and a mirror 300 placed on a linear bearing 230 which enables motion.

12. The device according to claim 10 or 11, which additionally has an encoder 280, preferably magnetic or optical, preferably incremental or absolute, for measuring the position of the reference path.

13. The device according to claim 12, whose encoder is configured to measure the displacement of the reference path between measurement windows.

14. The device according to claim 12 or 13, whose encoder is magnetic, the magnetic tape of the encoder is placed on a moving carriage along with the mirror 300, and the encoder sensor is placed on a frame shared with the rail of the linear bearing 230.

15. The device according to any of the claims from 10 to 14, which is a portable device.
